Europäisches Patentamt

European Patent Office

Office européen des brevets

(11) Publication number: **0 330 451**
A2

# EUROPEAN PATENT APPLICATION

(21) Application number: 89301731.9

(22) Date of filing: 22.02.89

(51) Int. Cl.⁴: **C 12 N 15/00**
C 12 P 21/02, C 12 N 1/20

(30) Priority: 22.02.88 JP 37453/88

(43) Date of publication of application:
30.08.89 Bulletin 89/35

(84) Designated Contracting States: **CH DE FR GB LI**

(71) Applicant: **TOA NENRYO KOGYO KABUSHIKI KAISHA**
**1-1 Hitotsubashi, 1-Chome Chiyoda-Ku**
**Tokyo 100 (JP)**

(72) Inventor: **Suzuki, Masanori**
**2-11D-101, Bishitsurugaoka Ohi-machi**
**Iruma-gun Saitama (JP)**

**Maki, Noboru**
**1-4-6, Nishitsurugaoka Ohi-machi**
**Iruma-gun Saitama (JP)**

**Yagi, Shintaro**
**Green Park Asashigaoka 101 4-8-8, Asashigaoka**
**Asaka-shi Saitama (JP)**

(74) Representative: **Bizley, Richard Edward et al**
**BOULT, WADE & TENNANT 27 Furnival Street**
**London EC4A 1PQ (GB)**

(54) A cDNA coding for human normal serum albumin a, and a process for production of the albumin.

(57) A cDNA coding for human normal serum albumin A; an expression vector comprising the cDNA coding for human normal serum albumin A; a host transformed with the expression vector comprising the cDNA coding for human normal serum albumin A; and a process for the production of the human normal serum albumin A comprising the steps of culturing a host transformed with an expression vector comprising a cDNA coding for the human normal serum albumin to express the protein alone or in a form of a fused protein with another protein, and obtaining the human normal serum albumin A.

**Description**

## A cDNA CODING FOR HUMAN NORMAL SERUM ALBUMIN A, AND A PROCESS FOR PRODUCTION OF THE ALBUMIN

The present invention relates to a process for the production of human normal serum albumin A by a recombinant DNA technique, and a gene therefor. According to the present invention, a large amount of human normal serum albumin A free of infection by pathogens such as hepatitis B virus, and AIDS virus HTLV, can be produced at a low cost.

Human serum albumin is a plasma protein synthesized in the liver, and plays an important role in an organism: It serves in the plasma to maintain osmotic pressure; binding various substances such as fatty acids, metal ions such as $Cu^{2+}$, $Ni^{2+}$, bile bilirubin, various drugs and water soluble vitamins and the like, to transport same to target organs thereof; and as a source of amino acids provided to tissues. On the basis of such actions, a large amount of human serum albumin is used to treat patients suffering from hemorrhagic shock and hypoalbuminemia generated by a reduced synthesis of albumin due to hepatocirrhosis, or by burns or nephritis.

An amino acid sequence of human normal serum albumin A is known on the basis of an amino acid analysis of natural human serum albumin, and further, cDNAs coding for human serum albumin are known. However, amino acid sequences of such polypeptides encoded by the known cDNAs are not completely the same as an amino acid sequence of human normal serum albumin A present in most human population. For example, cDNA described by Dugaiczyk et al., Proc. Natl. Acad. Sci. USA, 79, 71 - 75 (1982) encodes Gly as the 97th amino acid, although Glu is in human normal serum albumin; cDNA described by Lawn et al., Nucleic Acids Res. 9, 6103 - 6114 (1981) encodes Lys as the 396th amino acid, although Glu is in human normal serum albumin; cDNA described by Mariotti et al., Protides Biol. Fluids Proc. Colloq., 33, 177 - 179 (1985) encodes Thr as the 92nd amino acid, although Ala is in human normal serum albumin, and Met as the 381st and 462nd amino acid, although Val is in human normal serum albumin at bath positions; and cDNA described in Japanese unexamined Patent Publication No. 58-150517 encodes Ser as the 369th amino acid, although Cys is in human normal serum albumin, and some amino acids have not been determined. A chromosomal DNA sequence coding for human normal serum albumin A is described by Minghetti et al., J. Biol. Chem. 261, 6747 - 6757 (1986).

Accordingly the above-mentioned cDNAs cannot be used to produce a protein having the same amino acid sequence as human normal serum albumin A.

However, when serum albumin having an amino acid sequence different from that of normal serum albumin is administered to a human, it may exhibit antigenicity and may not exhibit the normal functions of serum albumin, or may have short life time in the blood. Therefore, there is a strong demand for the obtaining of cDNA correctly encoding an amino acid sequence of human normal serum albumin to produce the human normal serum albumin by a recombinant DNA technique.

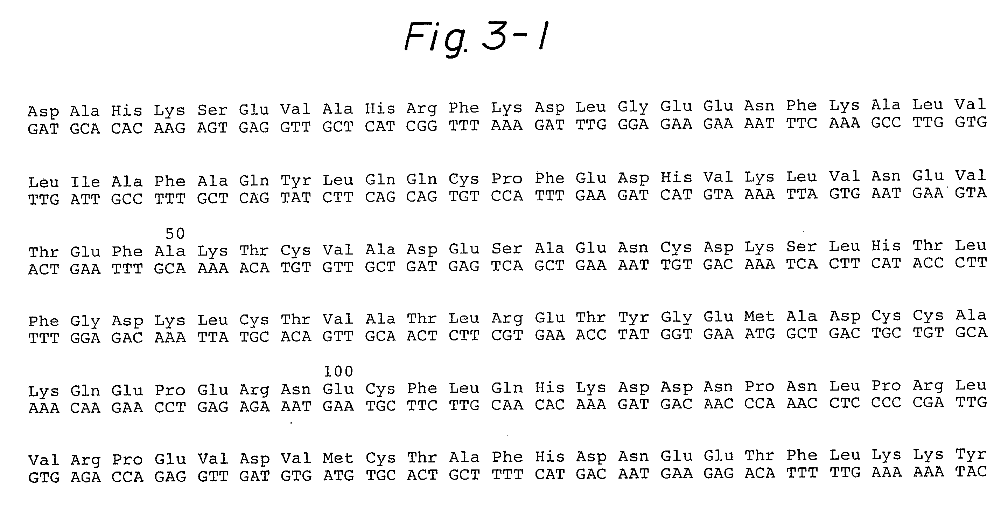

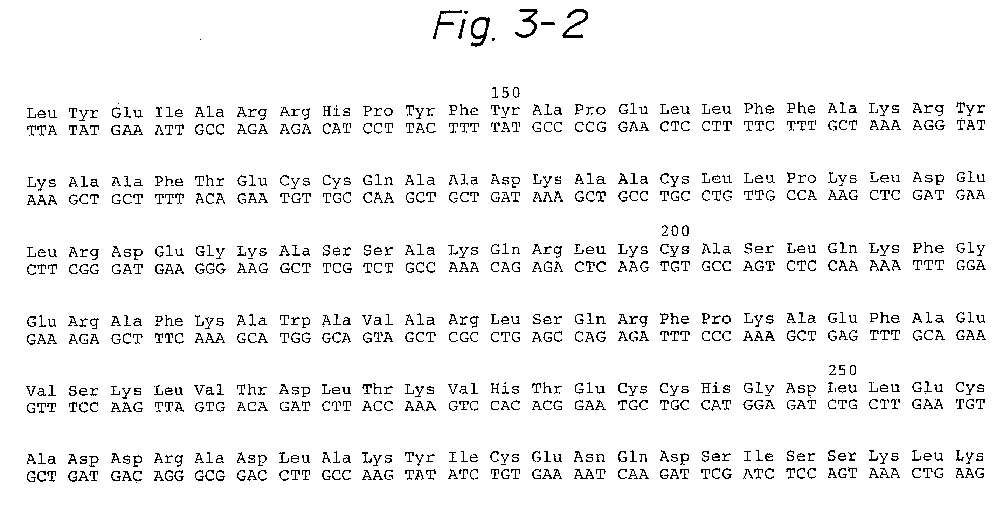

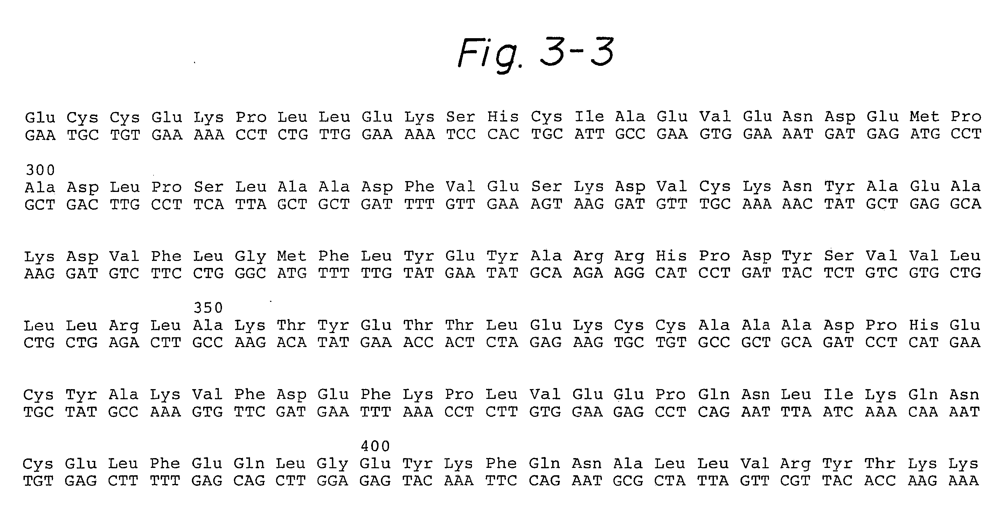

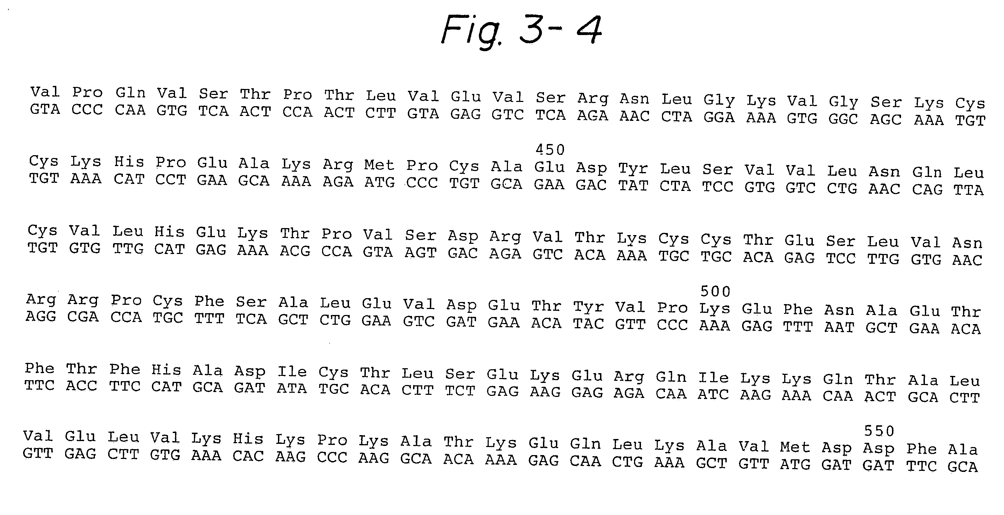

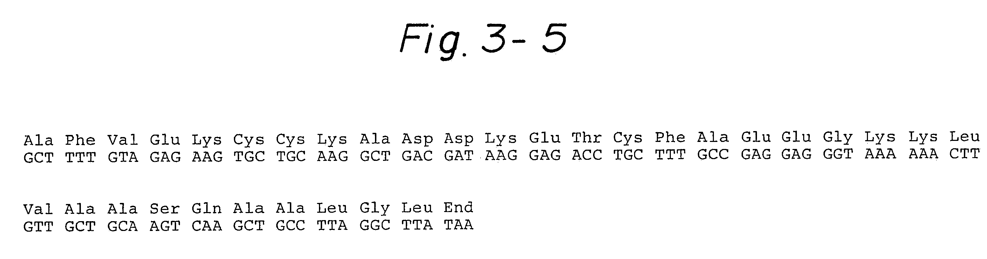

Accordingly, the present invention provides a cDNA coding for human normal serum albumin A having an amino acid sequence represented in Figures 3-1 to 3-5.

Moreover, the present invention provides an expression plasmid comprising a cDNA coding for human normal serum albumin A having an amino acid sequence represented in Figures 3-1 to 3-5.

The present invention also provides a host transformed with an expression plasmid comprising a cDNA coding for human normal serum albumin A having an amino acid sequence represented in Figures 3-1 to 3-5.

The present invention still further provides a process for the production of human normal serum albumin A comprising the steps of culturing a host transformed with an expression plasmid comprising a cDNA coding for the human normal serum albumin A having an amino acid sequence represented in Figures 3-1 to 3-5, to express the protein alone or in a form of a fused protein with other protein, and obtaining the human normal serum albumin A.

In the drawings:-

Figure 1 shows restriction enzyme cleavage maps of a cDNA fragment (HSAcDNA) coding for an entire human normal serum albumin A of the present invention, as well as a cDNA fragment (HSA-IA) coding for 3′-terminal side and a cDNA fragment (HSA-II) coding for the 5′-terminal side;

Figs. 2-1 to 2-2 show a construction process of various plasmids related to the present invention;

Figs. 3-1 to 3-5 show a nucleotide sequence coding for an entire human normal serum albumin A of the present invention, and an amino acid sequence corresponding to the nucleotide sequence;

Fig. 4 shows a result of an electrophoresis of an expression product representing proteins reacted with an anti-human serum albumin antibody; and,

Fig. 5 shows a nucleotide sequence of three probes used to screen a cDNA library.

A cDNA coding for human normal serum albumin A can be obtained by screening a human cDNA library by a conventional procedure; for example, a human liver library prepared using phage λgt11 as a vector. Probes for screening the cDNA library can be designed on the basis of a known nucleotide sequence of cDNA coding for human serum albumin. Preferably, a combination of three probes coding for an N-terminal region, central region, and C-terminal region of the human serum albumin, respectively is used. To obtain DNA correctly coding for an entire human normal serum albumin A, conveniently, different cDNA fragments coding for different parts of human serum albumin A are selected and sequenced, and after confirming that they correctly encode corresponding parts of the human normal serum albumin, appropriate parts thereof are joined to form an entire cDNA. Where it is found that a part of a cDNA fragment does not correctly encode a corresponding part of an amino acid sequence of the human normal serum albumin A, the part of the cDNA not correctly encoding the amino acid sequence is replaced by a cDNA fraction which correctly encodes the amino acid sequence in question, to construct a correct entire cDNA. Where the construction of an entire cDNA from partial cDNA fragments is difficult, the part of DNA not obtained from cDNA can be supplemented by a synthetic double-stranded DNA fragment.

The cDNA coding for human normal serum albumin A by itself of the present invention can be expressed. Alternatively, the cDNA of the present invention can be joined with other DNA coding for other peptides to express the human normal serum albumin in the form of a fused protein. As a partner for such a fused protein, various peptides can be used, and as an example of the partner peptide, a signal peptide of E. coli alkaline phosphatase can be mentioned. Where the human normal serum albumin A is expressed as a fused protein, the signal peptide can be eliminated from the fused protein after the expression, to obtain the desired human normal serum albumin A.

To express the human normal serum albumin A as a fused protein, cDNA coding for the fused protein is inserted into an expression vector, which is then introduced into a host. As a host for the expression, eukaryotic cells such as animal cells, yeast cells, and bacterial cells can be used, and a vector is selected according to a host selected. An expression plasmid comprises expression control regions including a promoter and Shine-Dalgarno (SD) sequence, followed by cDNA coding for the human normal serum albumin.

As the promoter, a trp promoter, lac promoter, λ phage promoter such as $P_R$ or $P_L$, tufB promoter or rrnB promoter, or a hybride promoter constructed from said promoters, such as an tac promoter, can be mentioned. As the SD sequence, a DNA sequence corresponding to a sequence in mRNA complementary to the 3'-terminal nucleotide sequence of E. coli 16S RNA is known to be effective for a start of the translation. Alternatively, a completely complementary synthetic DNA fragment can be used as an SD sequence.

Transformation of a host such as E. coli with an expression plasmid can be carried out by a conventional procedure. A transformed host such as E. coli is cultured by a conventional procedure. When E. coli cells are grown to a predetermined cell concentration, it is induced to express the desired gene. The method of induction depends on promoter used, for example, a trp promoter is used and 3-indoleacrylic acid is added to the culture to induce the expression.

Where E. coli is used as a host, the desired protein is intracellularly accumulated. Therefore, to recover the desired protein, the cultured cells are collected, washed, resuspended in water or a buffer, and then disrupted. Since the desired protein is contained in an insoluble fraction, the insoluble fraction is collected by, for example, centrifugation or filtration, and if necessary, washed. Next, the recovered insoluble fraction is put into a protein solubilizing buffer such as a buffer containing sodium dodecyl sulfate and 2-mercaptoethanol to solubilize proteins.

Next, from the resulting solution containing a fused protein comprising the human normal serum albumin, the protein is recovered and purified by a conventional procedure. The fused protein can be cleaved by, for example, E. coli leader peptidase (signal peptidase I) in vitro, to obtain the desired human normal serum albumin A, by a procedure described by Zwizinski, C. and Wickner, W., J. Biol. Chem. 255, 7973 (1980).

Examples

The present invention will be further illustrated by, but is by no means limited to, the following examples.

Example 1. Screening of clones containing cDNA coding for human normal serum albumin A

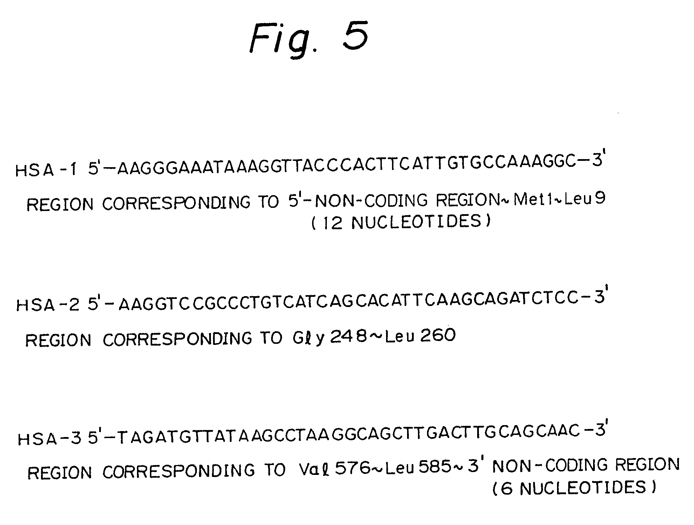

A human liver cDNA library constructed using a vector phage λgt11 commercially available from Clontech, U.S.A. was used to select clones containing a cDNA fragment coding for human normal serum albumin A by plaque hybridization. The λgt11 recombinant phage of the library was infected to E. coli Y1090, which was then plated on an LM agar medium to form $5.5 \times 10^5$ transformant plaques. Recombinant DNAs in the plaques were transfered onto membrane filters (Hybond-N; Amersham), and screened using three synthesized oligonucleotide probes labeled with $^{32}P$ (specific radioactivity $\geq 10^7$ cpm/μg) by a method of Benton and Davis, Science, 196, 180 - 182 (1977). These three probes are a probe HSA-1 corresponding to a 5'-non-coding region and a 5'- coding region starting 12 base-pairs upstream from ATG start codon and ending at in a codon for 9th amino acid leucine; a probe HSA-2 coding for 248th glycine to 260th leucine; and a probe HSA-3 comprising a 3'-terminal coding region and a 3'-terminal non-coding region starting with a codon for 576th valine and ending 9 nucleotides downstream from the C-terminal leucine codon, all described by Lawn et al., Nucleic Acids Res. 9, 6103 - 6114 (1981). The nucleodide sequence used as probes were on the complementary or negative strand. The nucleotide sequences of these three probes are shown in Fig. 5. These oligonucleotide probes were synthesized by an automatic DNA synthesizer, and labeled using [γ-$^{32}P$] ATP and polynucleotide kinase. Among 200 λgt11 clones which gave a positive signal with the probe HSA-2, from 4 clones, DNA was prepared by a method of Blattner et al., Science, 202, 1279-1284 (1978), and digested with EcoRI, and a Southern blot of the digested product was allowed to hybridize with the probe HSA-2 by a method of Southern, J. Mol. Biol. 98, 503 - 517 (1975). DNA fragments having a size of 1.8 Kb, 1.4 Kb, and 1.3 Kb, respectively, were hybridized with the probe HSA-2. Among these, DNA fragments of 1.8 Kb and 1.3 Kb were subcloned in vector pUC19, and these subclones were subjected to colony hybridization using probes HSA-1 and HSA-3, by a method of Grunstein and Hogness, Proc. Natl. Acad. Sci. USA, 72, 3961 - 3965 (1975).

As a result, a clone λgt11 (HSAI-A) which was hybridized with only HSA-3 was obtained. DNA in this clone was digested with various restriction enzymes, and the resulting DNA fragments were inserted into phage vectors M13mP18 and M13m19 RF DNA, and a nucleotide sequence of the DNA was determined by a dideoxy chain termination method of Sanger, F., Nicklen, S. and Coulson, A.R. Proc. Natl. Acad. Sci, USA, 74, 5463 - 5467 (1977).

On the other hand, among the clones which gave a positive signal in plaque hybridization of λgt11 clones using the HSA-2 probe, 20 clones were subjected to plaque hybridization using the HSA-1 probe, and a positive clone λgt11 (HSA-II) was obtained. From this clone, phage DNA was prepared and digested with EcoRI. The digestion product was subjected to Southern hybridization using the HSA-I probe, and a DNA fragment of 1.25 Kb designated HSA-II was found to hybridize with the HSA-I probe. A nucleotide sequence of this DNA fragment was determined by a dideoxy chain termination method. The HSA-II did not hybridize with the HSA-3 probe.

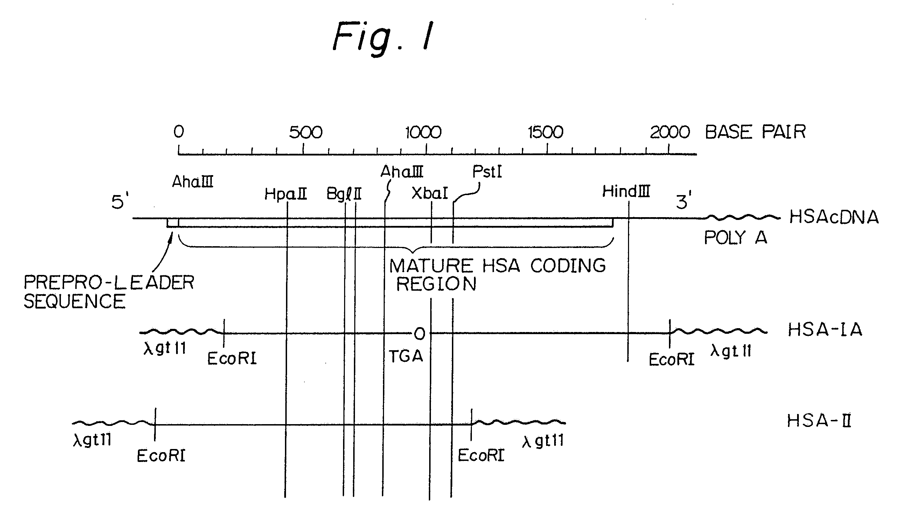

As a result, it was found that the HSA-II lacks a DNA portion coding for the C-terminal portion of human serum albumin, and the HSA-I-A lacks a DNA portion coding for the N-terminal portion of human serum albumin and containing an opal codon TGA as a stop codon in place of the codon TCA coding for 304th serine. Restriction enzyme cleavage maps of these DNA fragments are shown in Fig. 1. In these maps, exact positions of restriction enzyme recognizing sites were obtained from a finally determined nucleotide sequence.

As seen from Fig. 1, the HSA-I-A and HSA-II can be cleaved at an appropriate site and rejoined at the corresponding site to construct cDNA correctly coding for a full length of a precursor protein of human normal serum albumin joined with a signal peptide and/or prosequence. An amino acid sequence of a precursor of human serum albumin, and a mature protein (lacking prepro sequence) encoded by the cDNA thus constructed, completely conforms to that of human normal serum albumin A present in serum of most human population. The present cDNA is clearly different from other cDNAs disclosed in other patent documents and scientific articles in which the cDNA encodes amino acid sequences different from that of human normal serum albumin A. Moreover, the cDNA of the present invention coding for a precursor of the human normal serum albumin A codes for the same amino acid sequence as that coded by the human chromosomal gene described by Minghetti et al., J. Biol. Chem. 261, 6747 - 6757 (1986), although the third nucleotide in two codons is different without any amino acid substitution. Table 1 shows the difference between amino acid sequences encoded by cDNA previously reported, chromosomal DNA and cDNAS of the present invention, respectively, as well as an amino acid sequence of human normal serum albumin purified from human serum.

## Table 1

Difference between amino acid sequences encoded
by human serum albumin cDNA, and by human
chromosomal gene, respectively, and amino
acid sequence of human normal serum
albumin A purified from human serum

| DNA or Protein (Reference) | | Position of amino acid residues | | | | | |
|---|---|---|---|---|---|---|---|
| | | 92 | 97 | 369 | 381 | 396 | 462 |
| Chromosomal DNA | (1) | Ala | Glu | Cys | Val | Glu | Val |
| cDNA-1 | (2) | Ala | Gly | Cys | Val | Glu | Val |
| cDNA-2 | (3) | Ala | Glu | Cys | Val | Lys | Val |
| cDNA-3 | (4) | Thr[1] | Glu | Cys | Met | Glu | Met |
| cDNA-HSA-A | (5) | Ala | Glu | Cys | Val | Glu | Val |
| Serum Protein-1 | (6) | Ala | Glu | Cys | Val | Glu | Val |
| cDNA-4 | (7) | –[2] | – | Ser | Val | Glu | – |

1)  Underlined amino acids are different from those of human normal serum albumin.

2)  The amino acid and nucleotide are not described.

### Reference

(1) Minghetti et al., J. Biol. Chem. 261, 6747 – 6757 (1986)

(2) Dugaiczyk et al., Proc. Natl. Acad. Sci. USA 79, 71 – 75 (1982)

(3) Lawn et al., Nucleic Acids Res. 9, 6103 – 6114 (1981)

(4) Marisitti et al., Protides Biol. Fluids Proc. Colloq., 33, 177 - 179 (1985)

(5) Present invention

(6) Takahashi et al., Proc. Natl. Acad. Sci.
USA 84, 4413 - 4417 (1987)

(7) Japanese Unexamined Patent Publication
58-150517

Example 2. Construction of plasmid pUC.phoA

A plasmid pUC.phoA containing a synthetic DNA fragment coding for signal peptide of E. coli alkaline phosphatase was constructed as follows.

A DNA fragment having the following nucleotide sequence coding for signal peptide of E. coli alkaline phosphatase was constructed from chemically synthesized oligonucleotide fragments.

```
EcoRI                                          30
  AA TTC ATG AAA CAA AGC ACT ATT GCA CTG
     G TAC TTT GTT TCG TGA TAA CGT GAC
       Met Lys Gln Ser Thr Ile Ala Leu
                                          60
GCA CTC TTA CCG TTA CTG TTT ACC CCT GTG
CGT GAG AAT GGC AAT GAC AAA TGG GGA CAC
Ala Leu Leu Pro Leu Leu Phe Thr Pro Val
           NaeI
ACA AAA GCC GGC G
TGT TTT CGG CCG C TT A A
Thr Lys Ala
             HPaII EcoRI
```

The EcoRI sites of both ends of the DNA fragments were provided to insert the fragment into the EcoRI site of a pUC series plasmid, the HpaII site was provided to fuse it with the HSA-A mature gene, and the NaeI site was provided to cleave the DNA fragment at a position immediately downstream of a codon for the last amino acid (the 21th alanine) of the signal peptide to make a blunt end, to which a DNA coding for the mature protein can be directly fused. Two DNA fragments each consisting of 72 nucleotides were synthesized using an automated DNA synthesizer (Applied Biosystems, Model 380B) by the phosphamidite method developed by Matteucci, M. D. and Caruthers, M. H., Tetrahedron Letters 21, 719 (1980). The synthesized DNA (21 pmoles) was phosphorylated at the 5'-end thereof by treatment with 6 units of T4 polynucleotide kinase (Takara Shuzo) at 37°C for 60 minutes in 50 µl of a solution containing 50 mM Tris-HCl (pH 7.6), 10 mM MgCl2 , 5 mM dithiothreitol and 0.2 mM ATP.

Each of the reaction mixtures (50 µl) containing a different 5'-phosphorylated DNA were mixed to make 100 µl, and the mixture was heated in a water bath at 100°C and allowed to cool, to anneal the DNAs. To improve the efficiency of insertion of the annealed phosphorylated DNA into plasmid pUC19, after cleaving the plasmid pUC19 with EcoRI, phosphate groups present at the 5'-ends of the cleaved DNA strands were eliminated to prevent a rejoining of the cleaved plasmid from rejoining during ligation. Namely, 1 µg of pUC19 was treated with 8 units of EcoRI (Nippon gene) at 37°C for 60 minutes in 20 µl of a solution containing 50 mM NaCl, 100 mM Tris-HCl (pH 7.5) and 7 mM MgCl2 , to obtain a linearized vector DNA. The reaction mixture was heated at 90°C for 5 minutes to inactivate the enzyme, and to the mixture, 38 µl of water and 1 unit of bacterial alkaline phosphatase was added to make a total volume 60 µl. The reaction mixture was incubated at 37°C for 60 minutes, the mixture was extracted with phenol, and the resulting aqueous phase was treated with ethanol to precipitate DNA, which was then lyophilized to be used in the next step.

The dephosphorylated linearized vector pUC19 (30 ng) thus prepared and 10 ng of the phosphorylated double-stranded DNA prepared as described above were treated with 2.8 units of T4 DNA ligase (Takara Shuzo) at 15°C for 4 hours in 30 µl of a solution containing 66 mM Tris-HCl (pH 7.6), 6.6 mM MgCl2, 10 mM

dithiothreitol and 1 mM ATP to obtain a plasmid.

Competent E. coli cells to be transformed were prepared by a calcium phosphate method of Mandel, M. and Higa, A., J. Mol. Biol. 53, 159-162 (1970). Namely, E. coli TB-1 was cultured overnight in LB medium containing 10 g tryptone, 5 g yeast extract, and 10 g NaCl in $1\ell$ of water (pH 7.4), the culture was diluted 100-fold with the same medium, and culturing was carried out at 37°C with shaking until an $OD_{600}$ value reached 0.6. The culture (1.5 ml) was centrifuged at 5000 rpm for 5 minutes to collect cells, the cells were then suspended in 750 μl of 50 mM $CaCl_2$, and after resting on ice for 20 minutes, the suspension was centrifuged to collect the cells. The resulting pellet was resuspended in 100 μl of 50 mM $CaCl_2$, and the above-mentioned DNA ligase reaction mixture was added to the suspension, and the mixture was maintained on ice for 40 minutes. After incubation at 42°C for one minute, 1 ml of LB medium was added to the mixture, which was then incubated at 37°C for 30 minutes. The incubated suspension (0.1 ml) was spread on an X-Gal agar medium prepared by dissolving 155 mg of 5-bromo-4-chloro-3-indolyl-β-galactoside, 10 g of tryptone, 8 g of NaCl and 12 g of agar in 1 $\ell$ of water and adjusting the pH to 7.2, and incubated overnight at 37°C. Among colonies formed on the agar plate, white colonies were selected, transferred to a fresh agar medium, and cultured overnight. Cells on the agar plate were picked up and inoculated to LB liquid medium and cultured overnight. The culture (1.5 ml) was centrifuged to collect cells. The cells were subjected to mini-preparation of plasmid DNA by a conventional procedure described by Maniatis et al., Molecular Cloning: A laboratory Manual 1982. The resulting plasmid DNA was cleaved with appropriate restriction enzymes, for example, those which cleave restriction sites in the inserted synthetic DNA such as EcoRI, NaeI, HpaII, etc., or those which cleave restriction sites in the vector pUC19 such as PvuI, BglI, SspI etc., and the cleavage products were analyzed by agarose gel electrophoresis or polyacrylamide gel electrophoresis to determine a size of the inserted DNA. In this manner, a recombinant plasmid which contained a DNA insert having an appropriate size was identified. A DNA fragment containing this DNA insert was introduced again into M13mp phage DNA, and the nucleotide sequence thereof was determined by a dideoxy chain termination method of Sanger, F., Nicklen, S., and Corlson, A.R., Proc., Natl. Acad. Sci. U.S.A. 74, 5463-5467 (1977). A desired plasmid pUS•phoA was identified.

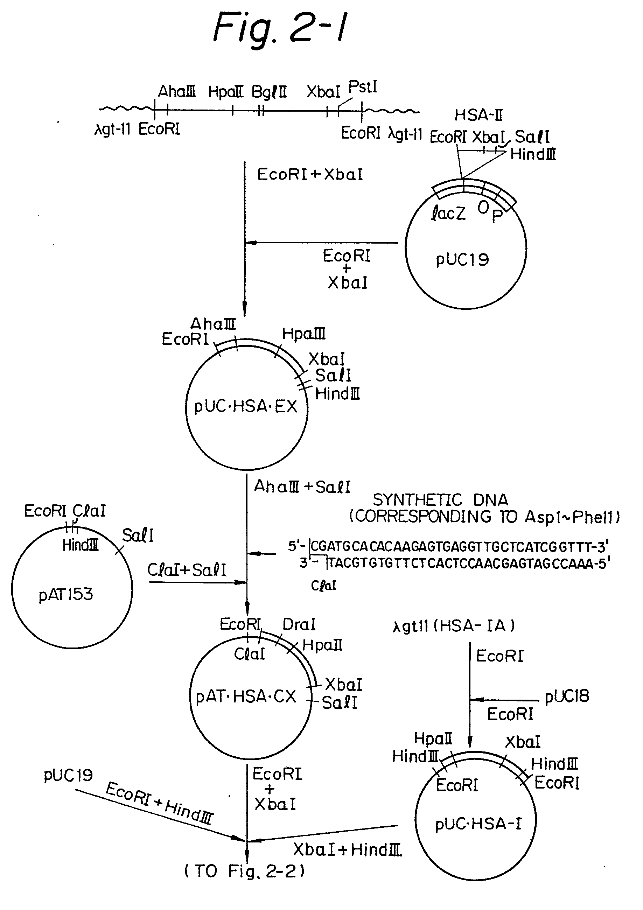

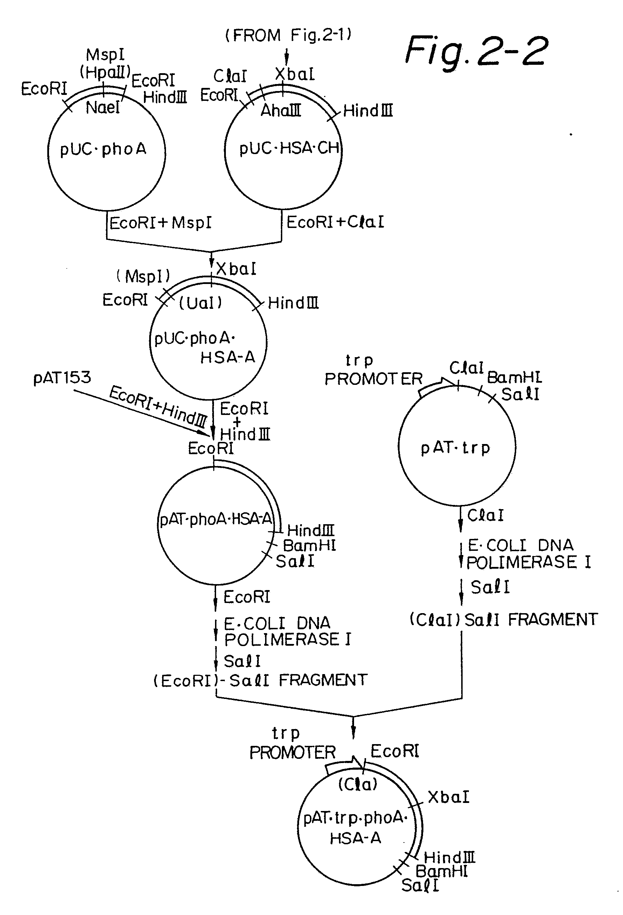

### Example 3. Construction of plasmid pUC-phoA-HSA-A (Figs. 2-1 to 2-2)

Plasmid pUC-phoA-HSA-A containing DNA coding for a fused protein comprising a signal peptide of E. coli alkaline phosphatase and human normal serum albumin A was constructed as follows.

A clone λgt11 (HSA-II) containing HSA cDNA derived from a human liver cDNA library was cleaved with EcoRI and XbaI to obtain a DNA fragment containing the cDNA. Plasmid pUC19 was cleaved with EcoRI and XbaI to obtain a larger DNA fragment. These DNA fragments were ligated together using T4 DNA ligase to construct a recombinant plasmid pUC-HSA-EX.

The plasmid pUC-HSA-EX was digested with AhaIII and SalI to obtain a smaller DNA fragment which encodes an amino acid sequence from 12th Lys to 356th Thr of human mature normal serum albumin A. To construct a gene coding for human mature normal serum albumin A, a DNA fragment corresponding to the 5'-portion of the mature albumin gene was prepared by annealing two chemically synthesized oligonucleotides. This DNA fragment has, at the 5'-terminal side thereof, an HpaII cleavage site and ClaI cleavage site to provide a cohesive end which can fuse with DNA coding for a signal peptide of alkaline phosphatase, and comprises codons coding for an amino acid sequence from the first Asp to 11th Phe. The annealed DNA fragment was phosphorylated at the 5'-end thereof using T4 ponucleotide kinase. On the other hand, a typical E. coli multicloning vector pAT 153 (Amersham; Twigg, A.J. and Sherratt, D., Nature, 283 216-218, 1980) was cleaved with ClaI and SalI, to obtain a larger DNA fragment. The above-prepared three DNA fragments were ligated using T4 DNA ligase to construct a recombinant plasmid pAT-HSA-CX. In this plasmid, DNA coding for the first Asp to 11th Phe is fused with DNA coding for the 12th Lys to 356th Phe. The plasmid pAT-HSA-CX was digested with EcoRI and XbaI to obtain a smaller DNA fragment coding for the first Asp to 356th Phe of the human normal serum albumin.

On the other hand, the phage λgt11 (HSAI-A) selected from the human liver cDNA library, as described above, was digested with EcoRI to obtain a DNA fragment containing a cDNA coding for the C-terminal half of the human normal serum albumin A. The DNA fragment was inserted to the EcoRI site of plasmid pUC18 to construct a recombinant plasmid pUC-HSA-1. This plasmid was digested with XbaI and HindIII to obtain a cDNA fragment containing the region coding for 358th Leu to the 585th carboxy terminal Leu and 3'-terminal non-coding region consisting of 62 nucleotides. On the other hand, a plasmid pUC18 was digested with EcoRI and HindIII to obtain a larger fragment. The above-prepared three DNA fragments were ligated using T4 DNA ligase to construct a recombinant plasmid pUC-HSA-CH containing an entire cDNA coding for human mature normal serum albumin.

A nucleotide sequence of cDNA coding for an entire amino acid sequence of human mature normal serum albumin A and a corresponding amino acid sequence are shown in Figs. 3-1 to 3-5.

To join the cDNA coding for human mature normal serum albumin A with DNA coding for a signal peptide of alkaline phosphatase (phoA), a plasmid pUC-HSA-CH was digested with EcoRI and ClaI to obtain a larger DNA fragment. A plasmid pUC-phoA was digested with EcoRI and MspI (recognizing the same sequence as that of HpaI) to obtain a smaller DNA fragment. These DNA fragments were ligated using T4 DNA ligase to construct plasmid pUC-phoA-HSA-A (Fig. 3), which contain DNA coding for a phoA signal peptide consisting of 21 amino acids fused to human mature normal serum albumin A. This plasmid was used to transform E. coli HB101.

Example 4. Construction of expression plasmid pAT-phoA-HSA-A

A plasmid pAT-phoA-HSA-A for expression of human normal serum albumin A was constructed as follows.

To express the above-mentioned gene in E. coli, the gene should be linked with an SD sequence responsible for an effective initiation of a translation and a promoter responsible for an effective initiation of a translation. In this example, a trp promoter and trpL SD sequence were used. A vector containing the trp promoter and trpL SD sequence is exemplified by plasmid ph-TNF (Ikehara et al., Chem. Pharm. Bulletin, in press) wherein the trp promoter and trpL SD sequence have been inserted in pBR322. However, to increase the copy number of a recombinant plasmid leading to a gene dosage effect, a plasmid based on plasmid pAT153 (Amersham; Twigg, A.J. and Sherratt, D., Nature, 283, 216-218, 1980) wherein replication poison sequence of pBR322 has been deleted, is preferably used. To this end, a plasmid ph•TNF was digested with PstI and ClaI to obtain a DNA fragment containing a trp promoter and trpL SD sequence. On the other hand, a plasmid pAT153 was digested with PstI and ClaI to obtain a larger DNA fragment. Next, these DNA fragments were ligated to construct a plasmid pAT-trp. The plasmid pAT-trp was cleaved at a unique ClaI site present downstream of the SD sequence, and resulting cohesive ends were filled in using E. Coli DNA polymerase I, and a resulting linearized plasmid was digested with SalI to obtain a larger fragment.

On the other hand, plasmid pUC-phoA-HSA-A was digested with EcoRI and HindIII to obtain a smaller DNA fragment containing phoA-HSA-A cDNA, which was then ligated to a larger EcoRI/HindIII double digest of pAT153 to construct a recombinant plasmid pAT-phoA-HSA. This plasmid was digested with EcoRI, and a resulting linearized plasmid was treated with E. coli DNA polymerase I to fill in the ends thereof, and cleaved with SalI to obtain a smaller DNA fragment containing phoA-HSA-A cDNA. This fragment was ligated with the DNA fragment prepared from plasmid PAT-trp, as described above, to construct a recombinant plasmid pAT-trp-phoA-HSA-A. This recombinant plasmid was used to transform E. coli HB101 and E. coli C600 to obtain E. coli HB101 (pAT-trp-phoA-HSA-A) and E. coli C600 (pAT-trp-phoA-HSA-A), respectively. E. coli C600 (pAT-trp-phoA-HSA-A) was deposited with the Fermentation Research Institute Agency of Industrial Science and Technology, 1-3, Higashi 1-chome, Tsukuba-shi, Ibaraki, Japan, on February 17, 1988 as FERM P-9874, and transferred to the international deposited under the Budapest Treaty as FERM BP- 2290 on February 17th, 1989.

Example 5. Production of fused protein

Fused protein comprising a signal peptide of E. coli alkaline phosphatase and human normal serum albumin A was produced using E. coli containing pAT-trp-phoA-HSA-A, as follows.

Culturing

E. coli C600m⁻r⁻ transformed with pAT-trp-phoA-HSA-A was inoculated to 5 ml of Luria broth (Bacto tryptone 1%, yeast extract 0.5%, NaCl 0.5%) supplemented with 25 µl ampicillin, and cultured for 18 hours at 37°C. A part of this culture (0.2 ml) was inoculated to 5 ml of M9-CA medium ($Na_2HPO_4$ 0.6%, $KH_2PO_4$ 0.3%, NaCl 0.5%, $NH_4Cl$ 0.1%, $CaCl_2$ 0.1 mM, $MgSO_4$ 2 mM, and casamino acid 0.8%) supplemented with 25 µg/ml ampicillin, and culturing was carried out at 37°C for 30 minutes. To the culture was added 20 µg/ml inducer 3-indole acrylic acid (IAA), and culturing was carried out at 37°C for an additional 5 to 7 hours.

Preparation of insoluble fraction

The culture prepared as described above was centrifuged at 7000 rpm for 5 minutes to collect cells. The precipitated cells were resuspended in 20% sucrose, 25 mM Tris-HCl (pH 7.5), 10 mM EDTA, 1 mM phenylmethanesulfonyl fluoride, and to the suspension was added egg white lysozyme to 0.2 mg/ml. The mixture was allowed to stand at 37°C for 15 minutes to digest the outer membrane, to obtain spheroplasts. The suspension was then cooled in ice, and centrifuged at 10000 rpm for 10 minutes to precipitate the spheroplasts. The spheroplasts were resuspended in a sucrose solution and disrupted in a Polytron homogenizer (dial: 8) in an ice bath. The homogenate was centrifuged at 15,000 rpm for 20 minutes at 4°C to obtain cell debris. The cell debris was resuspended in 25 mM Tris-HCL (pH 7.5), and the suspension was centrifuged at 15,000 rpm for 20 minutes. This operation was repeated once more to obtain a desired insoluble fraction.

SDS-polyacrylamide gel electrophoresis

1) Analysis of whole cellular protein

A part of the culture (0.5 ml) was centrifuged at 7000 rpm for 5 minutes to collect cells. The cells were suspended in 10 µl of SDS-sample solution (62.5 mM Tris-Hcl, pH 6.8, 2% SDS, 10% sucrose, 5% 2-mercaptoethanol, and the suspension was heated at 100°C for 5 minutes. This was subjected to electrophoresis on SDS-polyacrylamide gel (gel concentration, 10%) by a method of Laemmli, Nature (London), 227, 680-685 (1970).

2) Analysis of insoluble fraction

A portion of the insoluble fraction prepared as described above was diluted with the SDS-sample solution, and the suspension was heated at 100°C for 5 minutes to dissolve the insoluble proteins, and subjected to SDS-acrylamide gel electrophoresis.

3) Staining and destaining

After electrophoresis, the gel was dipped in a staining solution containing 0.25% Coomassie Brilliant Blue, 45% ethanol and 10% acetic acid for 30 to 60 minutes, and then in a destaining solution containing 5% methanol and 10% acetic acid in a destaining apparatus (BioRad, Model 555 type).

Western blotting and immunological detection

After finishing the SDS-PAGE, the gel was removed from the glass plate, and a nitrocellulose filter (Bio-Rad, Trans-blot ® ) and two 3 MM filter papers (Whatman) were impregnated with a blotting solution (0.3% Tris, 1.44% glycine and 20% methanol). On a pad previously impregnated with the blotting solution, the above-mentioned filter paper, gel, nitrocellulose filter, and filter paper were piled in this order, the upper filter paper was covered with the pad, and the whole was put in a blotting apparatus (TEFCO; Model: TC-808). The apparatus was filled with the blotting solution, and an electrophoresis was carried out at 200 mA for one hour.

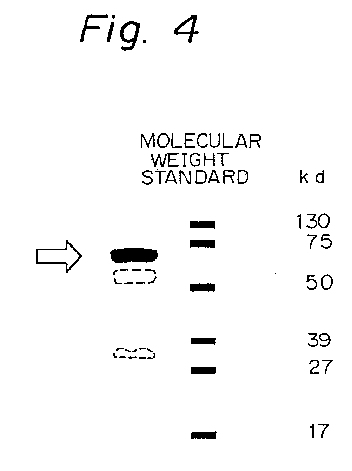

After finishing the electrophoresis, the nitrocellulose filter was peeled from the gel and treated in a TBS solution (25 mM Tris-HCl, pH 7.5, 0.5 M NaCl) for 10 minutes. The filter was then treated in a TBS solution containing 3% gelatin for 30 minutes, followed by treatment in TBS containing 0.025% Tween 20 (TTBS solution) for 5 minutes. This procedure was repeated. An IgG fraction of rabbit anti-human albumin serum (Cappel) was diluted 2000-fold with TTBS containing 1% gelatin, and the filter was dipped in this solution for 2 to 18 hours. The sheet was then transferred in TTBS and maintained therein for 5 minutes. This procedure was repeated twice. A horseradish peroxidase-labeled goat anti-rabbit IgG antibody (Bio-Rad) was diluted 3000-fold with TTBS containing 1% gelatin, and the filter was dipped in this solution for 2 hours. Next, the filter was washed twice with TTBS and once with TBS, for 5 minutes each. The filter was dipped in TBS containing 0.015% $H_2O_2$ , 0.05% HRP color development reagent (Bio-Rad) and 16.7% methanol for 15 minutes, and then dipped in water for 30 minutes. The band of substance which cross-reacted with human normal serum albumin A was colored deep purple on the filter (Fig. 4), and an expression product of the present invention having a molecular weight of 69,000 was detected.

**Claims**

1. A cDNA coding for human normal serum albumin A having an amino acid sequence represented in Figures 3-1 to 3-5.

2. A cDNA according to claim 1, having a nucleotide sequence represented in Figure 3-1 to 3-5.

3. An expression plasmid comprising a cDNA coding for human normal serum albumin A having an amino acid sequence represented in Figures 3-1 to 3-5.

4. An expression plasmid according to claim 4, wherein the cDNA has a nucleotide sequence represented in Figures 3-1 to 3-5.

5. A host transformed with an expression plasmid comprising a cDNA coding for human normal serum albumin A having an amino acid sequence represented in Figures 3-1 to 3-5.

6. A host according to claim 5, wherein the cDNA has a nucleotide sequence represented in Figures 3-1 to 3-5.

7. A process for production of human normal serum albumin A comprising the steps of culturing a host transformed with an expression plasmid comprising a cDNA coding for the human normal serum albumin having an amino acid sequence represented in Figures 3-1 to 3-5 to express the protein alone or in a form of a fused protein with another protein, and obtaining the human normal serum albumin A.

8. A process according to claim 7, wherein the cDNA has a nucleotide sequence represented in Figure 3-1 to 3-5.

Fig. 1

# Fig. 2-1

EP 0 330 451 A2

Fig. 2-2

# Fig. 3-1

```
Asp Ala His Lys Ser Glu Val Ala His Arg Phe Lys Asp Leu Gly Glu Glu Asn Phe Lys Ala Leu Val
GAT GCA CAC AAG AGT GAG GTT GCT CAT CGG TTT AAA GAT TTG GGA GAA GAA AAT TTC AAA GCC TTG GTG


Leu Ile Ala Phe Ala Gln Tyr Leu Gln Gln Cys Pro Phe Glu Asp His Val Lys Leu Val Asn Glu Val
TTG ATT GCC TTT GCT CAG TAT CTT CAG CAG TGT CCA TTT GAA GAT CAT GTA AAA TTA GTG AAT GAA GTA

                    50
Thr Glu Phe Ala Lys Thr Cys Val Ala Asp Glu Ser Ala Glu Asn Cys Asp Lys Ser Leu His Thr Leu
ACT GAA TTT GCA AAA ACA TGT GTT GCT GAT GAG TCA GCT GAA AAT TGT GAC AAA TCA CTT CAT ACC CTT


Phe Gly Asp Lys Leu Cys Thr Val Ala Thr Leu Arg Glu Thr Tyr Gly Glu Met Ala Asp Cys Cys Ala
TTT GGA GAC AAA TTA TGC ACA GTT GCA ACT CTT CGT GAA ACC TAT GGT GAA ATG GCT GAC TGC TGT GCA

                    100
Lys Gln Glu Pro Glu Arg Asn Glu Cys Phe Leu Gln His Lys Asp Asp Asn Pro Asn Leu Pro Arg Leu
AAA CAA GAA CCT GAG AGA AAT GAA TGC TTC TTG CAA CAC AAA GAT GAC AAC CCA AAC CTC CCC CGA TTG


Val Arg Pro Glu Val Asp Val Met Cys Thr Ala Phe His Asp Asn Glu Glu Thr Phe Leu Lys Lys Tyr
GTG AGA CCA GAG GTT GAT GTG ATG TGC ACT GCT TTT CAT GAC AAT GAA GAG ACA TTT TTG AAA AAA TAC
```

EP 0 330 451 A2

# Fig. 3-2

```
                                                     150
Leu Tyr Glu Ile Ala Arg Arg His Pro Tyr Phe Tyr Ala Pro Glu Leu Leu Phe Phe Ala Lys Arg Tyr
TTA TAT GAA ATT GCC AGA AGA CAT CCT TAC TTT TAT GCC CCG GAA CTC CTT TTC TTT GCT AAA AGG TAT


Lys Ala Ala Phe Thr Glu Cys Cys Gln Ala Ala Asp Lys Ala Ala Cys Leu Leu Pro Lys Leu Asp Glu
AAA GCT GCT TTT ACA GAA TGT TGC CAA GCT GCT GAT AAA GCT GCC TGC CTG TTG CCA AAG CTC GAT GAA

                                                             200
Leu Arg Asp Glu Gly Lys Ala Ser Ser Ala Lys Gln Arg Leu Lys Cys Ala Ser Leu Gln Lys Phe Gly
CTT CGG GAT GAA GGG AAG GCT TCG TCT GCC AAA CAG AGA CTC AAG TGT GCC AGT CTC CAA AAA TTT GGA


Glu Arg Ala Phe Lys Ala Trp Ala Val Ala Arg Leu Ser Gln Arg Phe Pro Lys Ala Glu Phe Ala Glu
GAA AGA GCT TTC AAA GCA TGG GCA GTA GCT CGC CTG AGC CAG AGA TTT CCC AAA GCT GAG TTT GCA GAA

                                                                 250
Val Ser Lys Leu Val Thr Asp Leu Thr Lys Val His Thr Glu Cys Cys His Gly Asp Leu Leu Glu Cys
GTT TCC AAG TTA GTG ACA GAT CTT ACC AAA GTC CAC ACG GAA TGC TGC CAT GGA GAT CTG CTT GAA TGT


Ala Asp Asp Arg Ala Asp Leu Ala Lys Tyr Ile Cys Glu Asn Gln Asp Ser Ile Ser Ser Lys Leu Lys
GCT GAT GAC AGG GCG GAC CTT GCC AAG TAT ATC TGT GAA AAT CAA GAT TCG ATC TCC AGT AAA CTG AAG
```

EP 0 330 451 A2

Fig. 3-3

```
Glu Cys Cys Glu Lys Pro Leu Leu Glu Lys Ser His Cys Ile Ala Glu Val Glu Asn Asp Glu Met Pro
GAA TGC TGT GAA AAA CCT CTG TTG GAA AAA TCC CAC TGC ATT GCC GAA GTG GAA AAT GAT GAG ATG CCT


300
Ala Asp Leu Pro Ser Leu Ala Ala Asp Phe Val Glu Ser Lys Asp Val Cys Lys Asn Tyr Ala Glu Ala
GCT GAC TTG CCT TCA TTA GCT GCT GAT TTT GTT GAA AGT AAG GAT GTT TGC AAA AAC TAT GCT GAG GCA


Lys Asp Val Phe Leu Gly Met Phe Leu Tyr Glu Tyr Ala Arg Arg His Pro Asp Tyr Ser Val Val Leu
AAG GAT GTC TTC CTG GGC ATG TTT TTG TAT GAA TAT GCA AGA AGG CAT CCT GAT TAC TCT GTC GTG CTG

        350
Leu Leu Arg Leu Ala Lys Thr Tyr Glu Thr Thr Leu Glu Lys Cys Cys Ala Ala Ala Asp Pro His Glu
CTG CTG AGA CTT GCC AAG ACA TAT GAA ACC ACT CTA GAG AAG TGC TGT GCC GCT GCA GAT CCT CAT GAA


Cys Tyr Ala Lys Val Phe Asp Glu Phe Lys Pro Leu Val Glu Glu Pro Gln Asn Leu Ile Lys Gln Asn
TGC TAT GCC AAA GTG TTC GAT GAA TTT AAA CCT CTT GTG GAA GAG CCT CAG AAT TTA ATC AAA CAA AAT

        400
Cys Glu Leu Phe Glu Gln Leu Gly Glu Tyr Lys Phe Gln Asn Ala Leu Leu Val Arg Tyr Thr Lys Lys
TGT GAG CTT TTT GAG CAG CTT GGA GAG TAC AAA TTC CAG AAT GCG CTA TTA GTT CGT TAC ACC AAG AAA
```

EP 0 330 451 A2

# Fig. 3-4

```
Val Pro Gln Val Ser Thr Pro Thr Leu Val Glu Val Ser Arg Asn Leu Gly Lys Val Gly Ser Lys Cys
GTA CCC CAA GTG TCA ACT CCA ACT CTT GTA GAG GTC TCA AGA AAC CTA GGA AAA GTG GGC AGC AAA TGT

                                                               450
Cys Lys His Pro Glu Ala Lys Arg Met Pro Cys Ala Glu Asp Tyr Leu Ser Val Val Leu Asn Gln Leu
TGT AAA CAT CCT GAA GCA AAA AGA ATG CCC TGT GCA GAA GAC TAT CTA TCC GTG GTC CTG AAC CAG TTA

Cys Val Leu His Glu Lys Thr Pro Val Ser Asp Arg Val Thr Lys Cys Cys Thr Glu Ser Leu Val Asn
TGT GTG TTG CAT GAG AAA ACG CCA GTA AGT GAC AGA GTC ACA AAA TGC TGC ACA GAG TCC TTG GTG AAC

                                                          500
Arg Arg Pro Cys Phe Ser Ala Leu Glu Val Asp Glu Thr Tyr Val Pro Lys Glu Phe Asn Ala Glu Thr
AGG CGA CCA TGC TTT TCA GCT CTG GAA GTC GAT GAA ACA TAC GTT CCC AAA GAG TTT AAT GCT GAA ACA

Phe Thr Phe His Ala Asp Ile Cys Thr Leu Ser Glu Lys Glu Arg Gln Ile Lys Lys Gln Thr Ala Leu
TTC ACC TTC CAT GCA GAT ATA TGC ACA CTT TCT GAG AAG GAG AGA CAA ATC AAG AAA CAA ACT GCA CTT

                                                               550
Val Glu Leu Val Lys His Lys Pro Lys Ala Thr Lys Glu Gln Leu Lys Ala Val Met Asp Asp Phe Ala
GTT GAG CTT GTG AAA CAC AAG CCC AAG GCA ACA AAA GAG CAA CTG AAA GCT GTT ATG GAT GAT TTC GCA
```

EP 0 330 451 A2

Fig. 3- 5

Ala Phe Val Glu Lys Cys Cys Lys Ala Asp Asp Lys Glu Thr Cys Phe Ala Glu Glu Gly Lys Lys Leu
GCT TTT GTA GAG AAG TGC TGC AAG GCT GAC GAT AAG GAG ACC TGC TTT GCC GAG GAG GGT AAA AAA CTT

Val Ala Ala Ser Gln Ala Ala Leu Gly Leu End
GTT GCT GCA AGT CAA GCT GCC TTA GGC TTA TAA

EP 0 330 451 A2

# Fig. 4

MOLECULAR
WEIGHT
STANDARD    k d

130
75

50

39

27

17

# Fig. 5

HSA-1 5'-AAGGGAAATAAAGGTTACCCACTTCATTGTGCCAAAGGC-3'

REGION CORRESPONDING TO 5'-NON-CODING REGION~Met1~Leu9
(12 NUCLEOTIDES)


HSA-2 5'-AAGGTCCGCCCTGTCATCAGCACATTCAAGCAGATCTCC-3'

REGION CORRESPONDING TO Gly248~Leu260


HSA-3 5'-TAGATGTTATAAGCCTAAGGCAGCTTGACTTGCAGCAAC-3'

REGION CORRESPONDING TO Val576~Leu585~3' NON-CODING REGION
(6 NUCLEOTIDES)

EP 0 330 451 A2